# EUROPEAN PATENT SPECIFICATION

(11) **EP 1 948 162 B1**
(45) Date of publication and mention of the grant of the patent: **22.05.2013**
(21) Application number: 06790501.8
(22) Date of filing: 20.10.2006
(51) Int. Cl.: A61K 31/395, A61K 36/48, C07D 487/00, A61P 25/00, A61P 25/22

(54) **PHARMACEUTICAL COMPOSITIONS CONTAINING ERYTHRINE MULUNGU DERIVATIVES AND PROCESSES FOR THEIR PRODUCTION**
PHARMAZEUTISCHE ZUSAMMENSETZUNGEN MIT ERYTHRIN-MULUNGU-DERIVATEN UND HERSTELLUNGSVERFAHREN
COMPOSITIONS PHARMACEUTIQUES CONTENANT DES DERIVES D'ERYTHRINE MULUNGU ET LEURS PROCEDES DE PRODUCTION

(30) Priority: 20.10.2005 BR PI0504517
(43) Date of publication of application: 30.07.2008
(73) Proprietor: Universidade Estadual Paulista Julio De Mesquita Filho-UNESP, 14190-001 Cerqueira César - SP (BR)
(72) Inventor: BOLZANI, Vanderlan da Silva, 14800-900 Araraquara - SP (BR); DE SOUZA, Ricardo Luis Nunes, 14801 -60 Araraquara - SP (BR); FLAUSINO JUNIOR, Otavio Aparecido, 14801 -125 Araraquara - SP (BR)
(74) Representative: Capasso, Olga
(86) International application number: PCT/BR2006/000220
(87) International publication number: WO 2007/045060

(56) References cited:
- WO-A1-2006/042389
- JP-A- 60 185 785
- ONUSIC G M ET AL: "EFFECTS OF CHRONIC TREATMENT WITH A WATER-ALCOHOL EXTRACT FROM ERYTHRINA MULUNGU ON ANXIETY-RELATED RESPONSES IN RATS" BIOLOGICAL & PHARMACEUTICAL BULLETIN (OF JAPAN), PHARMACEUTICAL SOCIETY OF JAPAN, TOKYO, JP, vol. 26, no. 11, 1 November 2003 (2003-11-01), pages 1538-1542, XP008094639 ISSN: 0918-6158
- VASCONCELOS SILVANIA M M ET AL: "Central activity of hydroalcoholic extracts from Erythrina velutina and Erythrina mulungu in mice" JOURNAL OF PHARMACY AND PHARMACOLOGY, ROYAL PHARMACEUTICAL SOCIETY OF GREAT BRITAIN, GB, vol. 56, no. 3, 1 March 2004 (2004-03-01), pages 389-393, XP008089907 ISSN: 0022-3573
- DATABASE MEDLINE [Online] ONUSIC G.M. ET AL.: 'Effects of chronic treatment with a water-alcohol extract from Erythrina mulungu on anxiety-related responses in rats', XP003012161 Database accession no. (NLM14600397) & BIOLOGICAL & PHARMACEUTICAL BULLETIN vol. 26, no. 11, 2003, pages 1538 - 1542
- DATABASE MEDLINE [Online] PAIVA M.A. ET AL.: 'Central activity of hydroalcoholic extracts from Erythrina velutina and Erythrina mulungu in mice', XP003012162 Database accession no. (NLM15025865) & THE JOURNAL OF PHARMACY AND PHARMACOLOGY vol. 56, no. 3, 2004, pages 389 - 393

## Description

### FIELD OF INVENTION

The present invention refers to molecules acting on the cholinergic, serotonergic and/or gabaergic systems. More specifically, the present invention refers to Erythrine byproducts useful in the preparation of anxiolytic medicines. Pharmaceutical compositions comprising said molecules and processes for preparing said pharmaceutical compositions are also provided.

### BACKGROUND TO THE INVENTION

*Erythrine mulungu* (Papilionaceae-Leguminoseae) is an arboreal plant (10 - 14 meters high) having red florescence, and grows in the semideciduous latifoliate forests of the Paraná basin and in scrubland regions, principally the western region of the State of São Paulo and Minas Triangle (LORENZI, 1992). The plant's bark is used by the local population as a tranquilizer and sedative. It is popularly known as mulungu, coral tree, coral mulungu, coral shrub (ethnic names include *capa-homem, suiná-suiná, tiriceiro* among others) (LORENZI, 1992). Eight varieties of Erythrine are found in Brazil: *E*. *mulungu, E. velutina, E. crita-galli, E. poeppigiana, E. fusca, E. falcata, E. speciosa* and *E. verna* (LORENZI, 1992).

Much work has been carried out to ascertain the fitochemical and pharmacological properties of other species of the *Erythrine* genus, which are also known for their popular use as a sedative, tranquilizer and also as a laxative, anti-inflammatory and anti-diuretic agent (GARÍN-AGUILAR *et al.,* 2000). Despite of the scarcity of studies on *E.* mulungu species, its extract is part of the composition of phytotherapic preparations commercially available in the Brazilian and international pharmaceutical market, however, without any standardization related to the product quality, being able to offer risks to the consumer's health.

### Fitochemistry

Interest in the study of the *Erythrine* species began in 1877 when Dominguez and Altamirano discovered that the pharmacological action of the seed extract of *E. americana* was similar to the effects of *d*-tubocurarine (substance extracted from *Chondodendron tomentosum)* (HARGREAVES *et al.,* 1974; HIDER *et al.,* 1986; GARÍN-AGUILAR *et al.,* 2000). From that point in time, investigations were carried out on the fitochemical and pharmacological properties of the extracts of different species of *Erythrine.* Years later, after confirmation of the pharmacological action displayed in the extracts of various species of *Erythrine,* research intensified towards isolating and identifying the alkaloids in plants of this kind (SARRAGIOTO *et al.,* 1981). Up until such time, pharmacological testing was performed on crude extracts. In 1937, Folkers and Major (1937) performed chemical investigations on the seeds of *E. americana* Mill. and isolated a crystalline alkaloid, erythroidine, which presented cholinergic activity similar to that of *d*-tubocurarine. Subsequent analyses (BOEKELHEIDE and GRUNDON, 1953; BOEKELHEIDE *et al.,* 1953) showed that erythroidine was a mixture of two isomeric alkaloids denominated α-erythroidine and β-erythroidine, the latter being responsible for the cholinergic activity, due to its capacity of antagonying peripheral nicotinic receivers (HARGREAVES *et al.,* 1974; HIDER *et al.,* 1986; GARÍN-AGUILAR, *et al.,* 2000).

After isolating α and β-erythroidine of *E. americana,* and the discoveries of its pharmacologic properties, there was great interest in the study of other species of *Erythrine* resulting in the isolation of new *E*. skeleton alkaloids (FOLKERS and KONIUSZY, 1940; FOLKERS *et al.,* 1944; BOEKELHEIDE and GRUNDON, 1953; BOEKELHEIDE *et al.,* 1953; TANDON *et al.,* 1969; ITO *et al.,* 1970; BARTON *et al.,* 1970; GHOSAL, 1970; GHOSAL *et al.,* 1971; ITO *et al.,* 1971; MIANA *et al.,* 1972; GHOSAL *et al.,* 1972 a,b; BARTON *et al.,* 1973; ITO *et al.,* 1973, a,b,c,d; GHOSAL and SRIVASTAVA, 1974; MILLINGTON *et al.,* 1974; GAMES *et al.,* 1974; ITO *et al.,* 1976; BARAKAT *et al.,* 1977; EL-OLEMY *et al*., 1978; AHMAD *et al*., 1979; TIWARI and MASSOD, 1979a,b; SARRAGIOTO *et al.,* 1981).

Clarification of the basic structure of the Erythrine alkaloids was achieved by degradation and synthesis (GRUNDON and BOEKELHEIDE, 1953; GRUNDON *et al.,* 1953; WEINSTOCK and BOEKELHEIDE, 1953; BOEKELHEIDE *et al.,* 1953). The presence of a spiroaminic skeleton was established in the structure of these alkaloids, which present the general formula below, facilitating the posterior identification of new compounds.

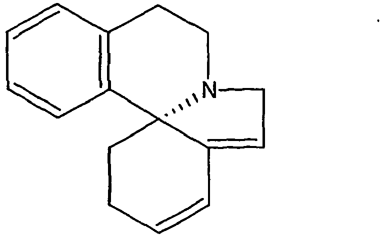

Clarification of this structure facilitated the subsequent identification of the new compounds. Currently, three types of Erythrine alkaloids are known: **1)** dienoids present a dienic system in rings A and B; **2)** alkaloids have a double bond Δ^{1,6} in ring A; **3)** a third group of Erythrine alkaloids includes: erysodienone, 3-desmethoxy erythratidinone, α-erythroidine and β-erythroidine. Some alkaloids of certain Erythrine species not presenting the *Erythrine* skeleton were also isolated, including: orientaline, *N*-Noorientaline, protosinomenine, *N*-Norprotosinomenine, isoboldine, erybidine, scoureline, coreximine, hypaforin, coline.

A fitochemical study of *E. mulungu* using ethanol extract prepared with the dried flowers isolated five alkaloids (erysothrina, *N*-erysothrina oxide, erythrartine, *N*-erythrartine oxide and hypaforin) and a terpenoid, fithol (SARRAGIOTO *et al.,* 1981; -SARRAGIOTO, 1981). Recent fitochemical studies have demonstrated that species of *Erythrine* are also rich in other classes of substances, such as flavanones, isoflavanones, isoflavones and pterocarpanes (DA-CUNHA *et al.,* 1998; TANAKA *et al.,* 1996, 1997a,b; 1998; 2001; OH *et al.,* 1999; YENESEW *et al.,* 2000; NKENGFACK *et al.,* 2001).

### Pharmacological activities

Among the principal pharmacological actions of *Erythrine* is its peripherical activity on the cholinergic system, which has been compared to the effects of d-tubocurarine (HARGREAVES *et al.,* 1974; HIDER *et al.,* 1986; GARÍN-AGUILAR, *et al.,* 2000). This effect was attributed to the alkaloid dihydro-β-erythroidine (DHBE), a nicotine-like antagonist receptor (HIDER *et al.,* 1986) isolated from *E. americana* (BOEKELHEIDE and GRUNDON, 1953; BOEKELHEIDE *et al.,* 1953) and *E*. *tholloniana* (CHAWLA *et al,* 1985). More recently, in an *in vitro* test, DHBE was characterized as a serotonergic 3 antagonist receptor (5-HT₃) (ELSELÈ *et al.,* 1993). The activity on the serotonergic system was also observed in another study (ROGER et *al.,* 2001), where the authors had demonstrated that the crude extract of *E. vespertilio* inhibited the calcium-dependent release of platelet serotonine, one of the main activities of the antagonists of 5-HT₃ receivers. However, the majority of the works is still carried out using crude extracts of different species of *Erythrine,* without the verification of the substances involved in the observed activities. As an example of the activities on the central nervous system anticonvulsivant, hypnosigenic, anaesthetic, sedative and anxiolytic effects can be cited (GHOSAL et al., 1972; HARGREAVES et al., 1974; RATNASOORIYA and DHARMASIRI, 1999; ONUSIC et *al.,* 2002, 2003).

A study into *E*. *velutina* demonstrated that acute treatment with hydroalcoholic extract decreased the activity of the mice in the open-field test (with doses of 250 and 500 mg/kg, oral intake), and also increased the period of sleep induced by pentobarbital and the period for the start of pilocarpine-induced convulsion (with doses of 500 and 1000 mg/kg, oral intake), indicating a depressive effect on the Central Nervous System (CABRAL *et al.,* 2000). Another work (GARÍN-AGULIAR *et al.,* 2000) revealed that acute treatment with the hexanic fraction of *E*. *americana* (3 mg/kg, i.p) decreased the aggressive behavior in male mice, similarly to diazepam. Recently, a study on the hydroalcoholic extract of *E. mulungu* (ONUSIC *et al.,* 2002) observed that acute treatment with a dose of 200 mg/kg (oral intake) presented an anxiolytic effect on mice in the inhibitory avoidance task in the elevated T maze test, comparable to that of the benzodiazepinic anxiolytic (BDZ), diazepam. ONUSIC and collaborators (2002) also observed the anxiolytic effect of *E*. *mulungu,* with the same dose, in the light/dark transition model, both in the number of transitions between the two model compartments as in the length of stay in the light compartment. Another work revealed that chronic treatment oral intake (9 days) with the extract of *E. mulungu* presented an anxiolytic effect, with doses of 50, 100 and 200 mg/kg, both in the inhibitory avoidance task, as in the escape from the open arms of the elevated T maze (ONUSIC *et al.,* 2003). In the light/dark transition model, the extract of *E*. *mulungu,* with a dose of 50 mg/kg, also presented an anxiolytic effect after chronic treatment for 14 days (ONUSIC *et al.,* 2003).

Despite these innumerous approaches, to-date there is no known report of the development of anxiolytic medicines made from standardized preparations is known, either in the form of crude extracts or purified fractions, of isolated active principles of *Erythrine,* or from the chemical synthesis thereof, nor any processes for preparing such medicines.

### SUMMARY OF THE INVENTION

It is an object of the present invention to supply a purified plant **fraction** rich in alkaloids for use as an anxiolytic agent, wherein said purified plant **fraction** contains at least 20% (w/w) of total erythrinic alkaloids and at least 10% (w/w) of erythravine and the plant is from the *Erythrine* genus. Preferably the plant is *Erythrina Mulungu.* In a further preferred embodiment *the* purified plant **fraction** is orally administered.

### DESCRIPTION OF THE FIGURE

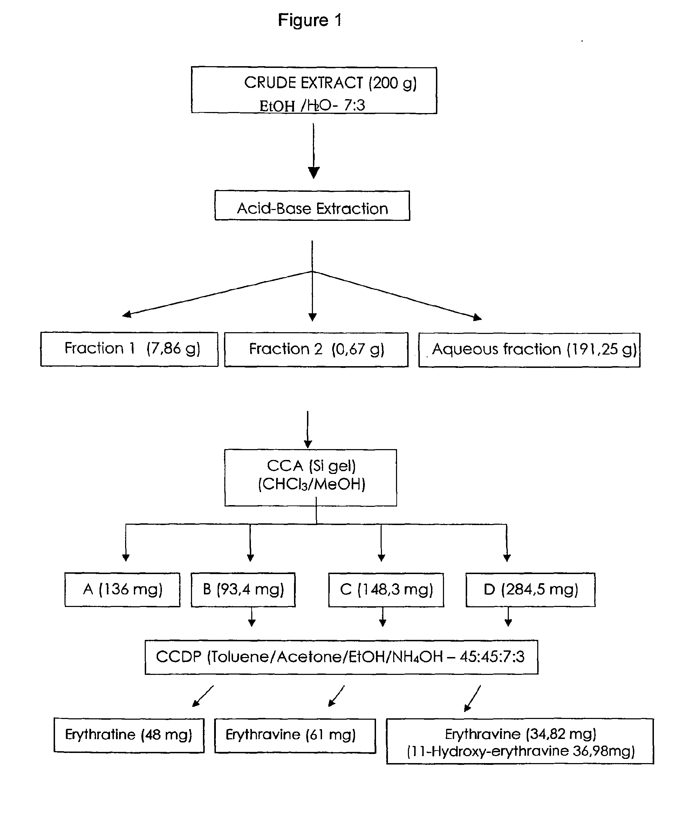

Figure 1 displays a general illustrative scheme of the stages of extraction and fractioning of the hydroalcoholic crude extract of *E*. *mulungu* and the isolation of the alkaloids erythrartine, erythravine and 11-OH-erythravine using the hydroalcoholic crude extract of *E. mulungu.*

### DETAILED DESCRIPTION OF THE INVENTION

For the purposes of this invention, pharmaceutical compositions" shall mean all and any composition containing an active principle, having prophylactic, palliative and/or curatives purposes, acting to maintain and/or restore the homeostasis, and may be administered topically, parenterally, enterally and/or intrathecally.

The pharmaceutical compositions referred to in this invention belong to the class of *Erythrine* byproducts and include 11-OH-erythravine, pharmaceutically acceptable isotherals, salts, byproducts and/or solvates thereof, optionally comprising erythrartine and/or erythravine isolated from crude extract or from the purified fraction of *E*. *mulungu* or chemical synthetics.

The therapeutic applicability of the compounds of the present invention was carried out in various stages. The experiments performed and the respective results are presented below merely as examples, and do not limit the scope of the attached claims.

### Example 1 - Phytochemistry

### Plant Collection and Extract preparation

Flowers of adult trees had been collected during the winter season, for example in the region of Araraquara (SP). The fresh vegetable material (6 kg) was submitted to extract by process of maceration with ethanol/water (EtOH/H₂O) (7:3) for 7 days. Next the extract was filtered and concentrated with the assistance of a rota-evaporator, resulting in 292 g of dry hydroalcoholic extract.

Its standardization in total alkaloids was carried out by volumetry of neutralization, according to pharmacopeic methodology, and established as containing, at least, 0,1% in alkaloids express in erytravine.

### Biomonitored Fractionation and Isolation of the Chemical Constituents

The acid-base extration was carried out in order to purify the crude extract and originate a fraction where the erythrinic alkaloid concentrations were optimized. To achieve this, the dry hydroalcoholic extract (120 g) was dissolved in an aqueous solution of acetic acid (10%) and submitted to liquid/liquid with chloroform extraction (CHCl₃).

The chloroform phase was separated from the aqueous phase and the solvent evaporated, resulting in fraction 1 (7.83 g). Next, the aqueous phase was alkalinized with ammonium hydroxide (NH₄OH) in a volume sufficient to attain a pH of between 9-10 and was again submitted to extraction with CHCl₃.

The chloroform phase was separated and the solvent evaporated, resulting in a purified fraction rich in alkaloids **(F2)** (670 mg). This fraction was evaluated by HPLC technique and was standardized as containg, at least, 20% in total alkaloids and 10% in erytravine. The F2 was also used in the pharmacologic test of the Labyrinth in high T (or elevated T maze).

### Example 2 - Chromatography, instrumentation and spectrometry

Degree solvents were used "for analysis".

For analytical thin-layer chromatography (CCD) of silica, CHCl₃/methanol (MeOH) (9:1) was used as the solvents system. The Dragendorf test was positive for alkaloids in **F2,** which was submitted to open-column chromatography (CCA) (5 cm in diameter and 15 cm high).

For the CCA (0,035-0,070 mm, φ 6 ηm) was used as silica stationary phase and CHCl₃/MeOH (10:0 - 8:2) as mobile phase. For separation, 670 mg of **F2** was used, and 101 fractions of approximately 20 ml were collected.

After having been submitted to analytical CCD, in mobile phase of CHCl₃/MeOH (7:1) and revealed by the Dragendorf assay, the 101 fractions were grouped in fraction A (**FA -** 136.2 mg) (1-27), fraction B (**FB** - 93.4 mg) (28-50), fraction C (**FC** - 148.3 mg) (51-69), fraction D (**FD** - 284.5 mg)(70-101).

To isolate and purify the alkaloids, preparative thin-layer chromatography (CCDP) was used, employing fluoresceine for the silica stationary phase (Merck) and toluene, acetone, ethanol and NH₄OH (45:45:7:3) for the mobile phase. From fraction B, eritrartine was isolated (48 mg); from fractions C and D, eritravine was isolated (61 and 34,82 mg, respectively); and from fraction D, 11-OH-eritravina was isolated (36,98 mg).

For the spectrometric analyses of the substances isolated from CCDP, a nuclear magnetic resonance (NMR) spectrometer Varian Unit was used, operating at 500 MHz. Deuterated chloroform (CDCl₃) was used as solvent.

### Example 3 - Pharmacology

### 3.1 - Animals Tests

Swiss mice weighing 25-35 g from the central animal laboratory of the São Paulo State University (UNESP/Araraquara) were used.

The animals were housed in groups of 10-12 animals, in polypropylene cages with wood shavings on the floor, with food and water available *ad libitum*]. The animal laboratory was maintained under constant temperature 22 ± 1°C, the lighting was controlled in 12-hour cycles from 7:00am to 7:00pm and the humidity was kept at between 50-60%.

### Example 4 - Rude standardized Extracts and rich purificated Fraction in alkalis, standard drug and Vehicle

Accordingly, lyophilized hydroalcoholic extract (50, 100, 200 and 400 mg/kg) was used, in addition to F2 (3, 6, 10, 17 and 30 mg/kg) and the alkaloids erythrartine, erythravine and 11-OH-erythravine (3 and 10 mg/kg), administered via oral intake by gavage. The standard drug used was Diazepam (DZP) in a dose of 2 mg/kg (via intraperitoneal, i.p).

All solutions were prepared on the day of the experiment with sodium chlorate 0.9% (0.9% NaCl) and sonicated for 15 minutes. The diazepam was dissolved in 0,9% NaCl and Tween-80.

The experiments were carried out between 11:00am and 5:00pm.

### Experimental Apparatus and Procedures

The Labyrinth in high T is made with transparent glass walls and wooden floor and consists of a closed arm (30 x 5 x 15 cm) perpendicularly linked to two open arms (30 x 5 x 0.25 cm), raised at 38.5 cm above the floor by a wooden support. In this test, five consecutive measures of inhibitory avoidance were performed (basal latency, avoidances 1, 2, 3 and 4) and one measure of escape from the open arms, with intervals of 30 seconds between each attempt. In the avoidance measures, the animals were placed in the distal section of the closed arm and the exit latency of this arm, on all four paws, towards the open arm was timed. In the escape measure, the animals were placed in the extremity of the right-hand open arm and the departure time from this arm was measured. The animals' maximum length of stay in the arms of the maze during these measures was 300 seconds. The apparatus was cleaned with ethanol 20% after testing each animal.

In order to avoid false positives or negatives, immediately after testing in the elevated T maze, the animals were submitted to the locomotive activity test in the arena.

The apparatus consists of a white polypropylene box with a rectangular base (40 x 48 cm), surrounded by 30cm high walls. The floor is subdivided into 30 squares (8 x 8 cm).

In this test, the animals were placed in the center of the box and their activity was video recorded for five minutes, for subsequent analysis of the number of crossings of the quadrant areas and number of stretch-attend postures (WALSH and CUMMINS, 1976).

### Analysis of Model Results

In order to determine the chemical structures of the isolated alkaloids it was used ¹H and ¹³C RMN spectrometry, as well as HMQC, HMBC and COSY bidimensional spectrometry. The results were compared with the information which already existed in the literature of *E*. alkaloids.

The results from the animal models were initially submitted to the Levene homogeneity test. The heterogeneous results were converted into a logarithmic scale and later analyzed statistically.

The results obtained from the Labyrinth in high T submitted to a two-way analysis of variance (ANOVA), with treatment being an independent factor and attempts being a dependent factor. When the effect of treatment proved to be significant, the data were analyzed using the one-way ANOVA followed by the Duncan *post hoc* test.

The results obtained with the arena were submitted to a one-way ANOVA followed by the Duncan *post hoc* test.

Amounts p ≤ 0,05 were considered to be significant results.

### Results

### 1) Phytochemical (Nuclear Magnetic Resonance - RMN)

Alkaloid **1** was isolated through CCDP with **FB.** Alkali **2** was isolated from **FC,** as well as from **FD.** From **FD** it was possible to isolate alkaloid **3** too.

The ¹H and ¹³C RMN spectrum in CDCl3 for substances **1, 2** and **3** (Table 2) showed the presence of characteristic signals of the *E*. alkali skeleton. In this regard, it was possible to identify signs of two singlets for the aromatic protons relating to hydrogens H-14 and H-17 and two singlets attributed to methoxyl hydrogens in the position of carbons C-15 and C-16. The presence of three signs of olefinic protons (broad singlet (sl), H-7; broad doublet (dl), H-1; two-doublet (dd), H-2), may be attributed to the dienic system hydrogens of the Erythrine skeleton.

Sarragioto *et al.* (1982) had reported resonances of C-1 and C-2 in δ 125.3 and δ 131.2 respectively. However, in the development of the present invention, the correlation between the chemical displacements of the HMQC bidimensional spectra demonstrated that these resonances occur at δ 131.5 and δ 125.5, respectively. Values of the chemical displacements and of the coupling constant are shown in Table 1.

**Table 1 - Chemical displacement measurements (δ) and coupling constants (J) of RMN (500MHz) of ¹H and ¹³C (in CD₃Cl) of erythrartine, erythravine and 11-OH-erythravine.**

| | ***Erythrartine*** | | ***Erythravine*** | | ***11-OH-Erythravine*** | |
|---|---|---|---|---|---|---|
| | *δ_{H} (J in Hz)* | *δ_{C}* | *δ_{H} (J in Hz)* | *δ_{C}* | *δ_{H} (J in Hz)* | *δ_{C}* |
| *1* | *5.94 (d; 10,5)* | *131.52* | *5.9 (d 10,0)* | *134.18* | *6.09(d; 15, 5)* | *135* |
| *2* | *6.52 (dd; 10,5; 2,5)* | *125.53* | *6.4 (dd; 10,0; 1,5)* | *124.96* | *6.56 (dd)* | *124.5* |
| *3* | *3.98 m* | *75.98* | *4.37 m* | *67.73* | *4.5 m* | *67.27* |
| *4* | *1.75 t* | *40.46* | *1.91 t* | *45.33* | *1.91t* | *43.9* |
| | *2.3 (dd; 19,5; 3,5)* | | *2.5 (dd; 11,0; 5,0)* | | *2.57dd* | |
| *5* | ------ | *66.30* | ------ | *67.11* | ------ | |
| *6* | ------ | *142.00* | ------ | *141.88* | ------ | *141.32* |
| *7* | *5.67 s* | *123.50* | *5.66 s* | *122.81* | *5.76s* | *124.45* |
| *8* | *3.81(d; 3,0)* | *58.71* | *3.58 d* | *56.45* | *3.99 d* | *58.78* |
| | *3.88 (dd)* | | *3.7 d* | | *3.93 d* | |
| *10* | *3.54 (dd; 14,0; 3,5)* | *50.92* | *3.05 (dd; 6, 0; 4,5)* | *43.40* | *3.10 dd* | *50.83* |
| | *3.07 (dd; 14,0; 4.5)* | | *3.50-3.53 m* | | *3.59 dd* | |
| *11* | *4.64 t* | *64.55* | *2.9 (dd, 10,0; 6,5)* | *23.76* | *4.74 t* | *63.69* |
| | | | *2.6-2.7 m* | | | |
| *12* | ------ | *128.32* | ------ | *126.01* | ------ | *?* |
| *13* | ------ | *129.68* | ------ | *130.61* | ------ | *?* |
| *14* | *6.91 s* | *108.68* | *6.64 s* | *111.52* | *6.71 s* | *108.36* |
| *15* | ------ | *148.28* | ------ | *147.19* | ------ | *141.32* |
| *16* | ------ | *148.52* | ------ | *147.77* | ------ | *148.76* |
| *17* | *6.77 s* | *110.33* | *6.83 s* | *109.16* | *6.93 s* | *110.36* |

### 1.1) Erythrartine

After the identification of the hydrogen characteristic signals of the *E*. skeleton (Table 2), it was noted that only the substance **1** showed the signal δ 3,24, which refers to three hydrogen of methoxyl located in C-3. According to the data found in literature for alkaloids 11-oxygen, the presence of one hydroxyl in C-11 was noted, considering the displacements as seen in the RMN spectrum of ¹H (broad triplet (t) in 4, 64) and of ¹³C (δ 64.55) and the correlations given with the HMQC test. In this regard, alkaloid **1** was identified as being erythrartine. Meanwhile, differently from the previous works, it was suggested an equatorial orientation for the group hydroxyl in C-11 from the values of the constants of coupling between H₁₀ₐ₋₁₁ₑ (J = 3.54 Hz) and H₁₀ₑ₋₁₁ₑ, (J = 3.07 Hz).

The chemical structure of the alkaloid erythrartine is showed below, isolated from the rude extract of the flowers of EM.

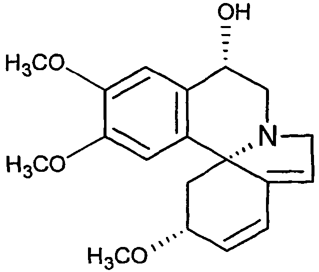

### 1.2) Erythravine

Differently from the previous substance, the ¹H RMN spectrum of substance **2** did not present signal of methoxyl in the C-3 position (Table 2). The multiplet (m) observed in δ 4,37 associated to the chemical displacement observed in δ 67.73 of the spectrum of ¹³C suggests the presence of one oxygen substitute in this position.

For the C-11 position the two-doublet observed in δ 2.9 (J_{H11ax-H10ax} = 10,0 Hz); J_{H11ax-H11eq} = 6,5 Hz) and the multiplet observed in the δ 2.65-2.69 region were attributed to the axial H-11 (ax) and to equatorial (eq), respectively. This substance was identified as erythravine, an erhitrinic alkaloid that had not been isolated in EM yet (figure 4).

The chemical structure of the alkaloid erhytravine is showed below, isolated from the rude extract of the flowers of EM.

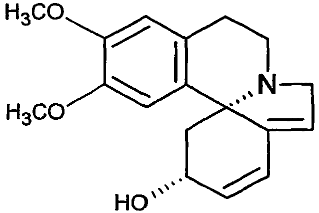

### 1.3) 11-hydroxy-erythravine (11-OH-erythravine)

In the NMR spectrum of ¹H for 11-OH-erythravine (Table 2), similarly to erythravine, there was not noted the sign of methoxyl hydrogens in the position C-3, but only a multiplet at δ 4.5 relating to a oxygenated substitute, attributed to the position C-3. In the same way as observed for erythrartine, the spectra of NMR ¹H and of ¹³C revealed chemical displacements at δ 4.74 (t) and δ 63.69, respectively, attributed to the presence of a hydroxyl in C-11. These results are being reported for the first time and, therefore, the substance **3** was recognized as being a new Erythrine alkaloid, and was named 11-hidroxi-erythravine (11-OH-erythravine).

**Table 2 - Effect (mean + EPM) of the acute treatment with the 11-OH-erhythravine in house mice submitted to the elevated T maze test.**

| ***Treatments*** | ***Inhibitory avoidances*** | | | | | ***Escape*** |
|---|---|---|---|---|---|---|
| | ***LB*** | ***E1*** | ***E2*** | ***E3*** | ***E4*** | |
| *SALINE* | *32,6±5,3* | *107,7±37,9* | *142,6±41,*7 | *165,0±37,7* | *232,8±35,5* | *21,8±3,1* |
| *DZP 2 mg*/*kg* | *24,6±4,1* | *15,3±3,8** | *12,25±1,6** | *22,3±7,7** | *24,2±7,3** | *19,0±3,2* |
| *11-OH 3 mg*/*kg* | *30,7±5,8* | *41,8±14,2* | *96,0±34,4* | *132,3±39,7* | *194,4±39,5* | *21,5±3,*7 |
| *11-OH 10 mg*/*kg* | *22,6±3,5* | *30,0±6,1** | *54,0±21,1* | *66,6±30,3** | *94,8±31,7** | *26,8±4,8* |

| | | | | | | |
|---|---|---|---|---|---|---|
| *p<0,05. | | | | | | |

The chemical structure of the alkaloid 11-OH-erythravine, isolated from the crude extract of *E*. *mulungu* flowers is presented below.

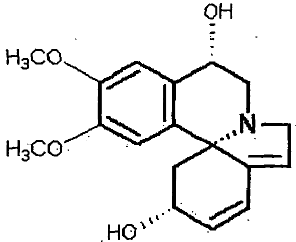

### 2) Pharmacology

### Crude extract

### 2.1) Labyrinth in high T (or Elevated T Maze Test)

As demonstrated in Table 3, the acute treatment with the standardized rude extract of EM harmed the performance of the animals in the inhibitory avoidance of the open arm of the labyrinth in high T.

The two-way ANOVA showed significant effect of treatment (F(5,49) = 5,44; p < 0,0001) and of the attempts (F(4,196) = 27,37; p < 0,0001), but not of the interaction between treatment and attempts (F(20,196) = 0,87, p = 0,628). The one-way ANOVA showed that there was significant difference among the groups of treatment in the basal latency (LB) (F(5,49) = 2,34; p = 0,056), avoidance 1 (E1) (F(5,49) = 3,97; p = 0,004), avoidance 2 (E2) (F(5,49) = 3,75; p = 0,005); avoidance 3 (E3) (F(5,49) = 3,39; p = 0,01) and avoidance 4 (E4) (F(5,49) = 2,62; p = 0,03).

In Table 3 the difference among the groups can be observed, when compared to the control, in accordance with the test post hoc of Duncan.

In the measure of escape of the open arm of the labyrinth in high T the one-way ANOVA showed that there was significant difference among groups (F(5,49) = 3,48; p = 0,009). The Duncan post-hoc test showed that this difference referred to a reduction of the latency of exit of the open arm towards the closed one presented by the group treated with the dozes of 400 mg/kg of EM (Table 3), when compared to the control group.

**Table 3 - Effect (mean + EPM) of the acute treatment with the crude extract of EM in house mouse submitted to Labyrinth in high T test (n = 9-10).**

| ***Treatments*** | ***Inhibitory avoidance*** | | | | | ***Scape*** |
|---|---|---|---|---|---|---|
| | **LB** | **E1** | **E2** | **E3** | **E4** | |
| *SALINE* | 54.4±5.8 | 64.4±18.2 | 148.6±33.3 | 205.8±37.5 | 216.9±30.6 | 20.6±3.3 |
| *DZP 2 mg*/*kg* | 25.4±4.1 | 19.1±4.0* | 25.3±7.0* | 59.8±30.9* | 91.3±40.3* | 28.4±7.1 |
| *EM 50 mg*/*kg* | 41.7±11.8 | 120.5±45.1 | 120.6±41.1 | 153.4±35.9 | 209.3±36.8 | 25.4±9.7 |
| *EM 100 mg*/*kg* | 42.4±17.7 | 57.7±20.72 | 76.1±34.1* | 126.2±45.6 | 138.5±43.6 | 32.1±19.3 |
| *EM 200 mg*/*kg* | 23.4±7.5 | 16.8±2.3* | 52.1±31.1* | 62.2±26.4* | 142.1±46.8 | 17.6±4.5 |
| *EM 400 mg*/*kg* | 26.4±5.1 | 45.±21.3 | 55.0±18.8* | 130.8±29.9 | 131.2+35.4 | 6.7±1.1* |

| | | | | | | |
|---|---|---|---|---|---|---|
| **p* < 0,05, Duncan Test. | | | | | | |

### 2.2) Locomotion Activity - Arena

The measurement of locomotion activity of animals at Arena has not been changed by the acute treatment with none of the doses of the crude extract of EM (table 4).

The ANOVA of one way did not show any significant differences between groups in the measure of the number of mixings (*F*(5,49) = 0.78; *p* = 0.56), neither in the measure of the number of surveys (*F*(5,49) = 1.90; *p* < 0.11).

**Table 4 - Effect (mean + EPM) of the acute treatment with the crude extract of EM on locomotive activity of in house mouse at Arena (n = 9-10).**

| ***Treatment*** | **Mixings** | **Raisings** |
|---|---|---|
| *SALINE* | *159.0±14.8* | *32.5±3.*7 |
| *DZP 2 mg*/*kg* | *167.4±23.6* | *27.33±2.3* |
| *EM 50 mg*/*kg* | *178.8±8.7* | *40.4±3.1* |
| *EM 100 mg*/*kg* | *166.1±4.4* | *37.8±2.5* |
| *EM 200 mg*/*kg* | *199.4±19.5* | *40.67±4.2* |
| *EM 400 mg*/*kg* | *167.3±18.3* | *38.4±5.37* |

### 3) Purified Fraction - riche in Eritrinics' alkaloids (F2)

### 3.1) Labyrinth in high T

The acute treatment with F2 harmed the acquisition of inhibitory avoidance in the open arm of the Labyrinth in high T, as demonstrated in table 5. The ANOVA of two ways showed a significant effect of treatment (*F*(4,40) = 7.00; *p* < 0.0001) and of attempts (*F*(4,160) = 11.18; *p* < 0.0001), but not for the interactions between treatment and attempts (F(16,160) = 1.48, *p* = 0.112). The ANOVA of one way showed that it happened a significant difference between treatment groups in LB *F*(6,69) = 2.7; *p* = 0.02), E1 (*F*(6,69) = 4.86; *p* < 0.0001), E2 (*F*(6,69) = 7.49; *p* < 0.0001); E3 (*F*(6,69) = 5.60; *p* < 0.0001) e E4 (*F*(6,69) = 7.83; *p* < 0.0001). The difference between groups in accordance with Duncan *post hoc* test can be observed in table 5.

The ANOVA of one way showed that did not occur a significant difference between groups (*F*(4,40) = 0.96; *p* = 0.46) in the measure of escape of the open arm of the Labyrinth in high T (table 5).

**Table 5 - Effect (mean + EPM) of the acute treatment with F2 in house mouse submitted to Labyrinth in high T test (n = 8-16).**

| ***Treatments*** | ***Inhibitory avoidance*** | | | | | ***Scape*** |
|---|---|---|---|---|---|---|
| | *LB* | *E1* | *E2* | *E3* | *E4* | |
| *SALINE* | *32.5±5.9* | *37.5±7.4* | *58.1±6.5* | *148.1±25.7* | *206.7±26.1* | *22.1±4.6* |
| *DZP 2 mg*/*kg* | *25.6±5.9* | *11.4±1.4** | *14.4±2.2** | *22.3±4.9** | *29.1±7.6** | *27.1±4.6* |
| *F2 3 mg*/*kg* | *21.7±3.2* | *32.0±13.2* | *18.5±2.12** | *109.3±34.9* | *91.0±26.7** | *20.1±4.1* |
| *F2 6 mg*/*kg* | *26.5±6.2* | *24.5±16.2* | *23.1±2.1** | *45.3±14.0** | *90.7±34.4** | *18.0±2.9* |
| *F2 10 mg*/*kg* | *24.6±4.3* | *29.5±7.9* | *26.1±6.7** | *44.5±12.2** | *74.1±29.2** | *23.8±6.8* |
| *F2 17 mg*/*kg* | *13.3±2.4* | *14.3±2.3** | *25.5±11.2** | *59.5±22.7** | *69.6±18.2** | *23.8±7.5* |
| *F2 30 mg*/*kg* | *56.1±21.8* | *65.0±34.0* | *78.6±38.7** | *85.1±35.6** | *121.2±41.2* | *30.1±8.4* |

| | | | | | | |
|---|---|---|---|---|---|---|
| * *p* < 0,05, Duncan Test | | | | | | |

### 3.2) Locomotion Activity - Arena

The measurement of locomotion activity of animals at Arena has not been changed by the acute treatment with none of the doses of F2 (table 6).

The ANOVA of one way did not show any significant differences between groups in the measure of the number of mixings (*F*(4,40) = 0.30; *p* = 0.87), neither in the measure of the number of raisings (*F*(4,40) = 0.81; *p* = 0.52).

**Table 6 - Effect (mean + EPM) of the acute treatment with purificated fraction rich in alkaloids (F2) on the locomotion activity of in house mouse at Arena (n=8-16).**

| ***Treataments*** | ***Mixings*** | ***Raisings*** |
|---|---|---|
| *SALINE* | *173.75±19.0* | *27.25±4.0* |
| *DZP 2 mg*/*kg* | *169.11±33.2* | *21.11±6.7* |
| *F2 3 mg*/*kg* | *164.54±12.5* | *31.18±4.5* |
| *F2 6 mg*/*kg* | *116.0±16.8* | *21.5±4.1* |
| *F2 10 mg*/*kg* | *145.00±9.8* | *31.1±3.5* |
| *F2 17 mg*/*kg* | *146.22±5.1* | *22.5±5.5* |
| *F2 30 mg*/*kg* | *161.00±16.4* | *26.00±2.8* |

### New Results of Isolated Alkloids

### 1) Erhytravine

The results obtained with Erythravine in house mouse submitted to the model of the clearly-dark transition are demonstrated in table 7.

The ANOVA of one way showed significant effect of treatment in the measure of time expense in the illuminated compartment (F(3,39) = 4.27; P = 0.01). The Duncan *post hoc* test showed that the time spend inside the illuminated compartment between animal groups treated with oral doses of 3 mg/kg and 10 *mg*/*kg* of erythravine and with DZP was expressively grater than between the control group. For the measure of the number of transitions between two compatments, ANOVA of one way did not show significant differences between groups (F(3,39) = 1.13; P = 0.34).

**Table 7 - Effect (mean + EPM) of the acute treatment with erythravine in house mouse submitted to the model of the clearly-dark transition (n = 10-12).**

| ***Treataments*** | ***Time spent in illuminated compartment*** | ***Number of transitions between two compartments*** |
|---|---|---|
| *SALINE* | *69.67±14.51* | *14.69±2.73* |
| *DZP (2 mg*/*kg)* | *144.20±17.52** | *20.10±2.93* |
| *ERI (3 mg*/*kg)* | *115.40±18.58** | *19.70±2.96* |
| *ERI (10 mg*/*kg)* | *112.00±10.95** | *19.80±1.32* |

| | | |
|---|---|---|
| * p < 0,05 versus Saline (Duncan *post hoc* Test) | | |

### 2) 11-OH-ERYTHRAVINE

In table 8 there are demonstrated the results obtained with 11-hydroxi-erhytravine transition's clearly-dark model.

The one-way ANOVA showed significant effect of the treatment in the measure of the time spent by the animals of the illuminated compartment (F(3,38) = 3.14; P < 0.05) and also for the number of transitions between two compartments (F(3,38) = 3.36; P < 0.05). The Duncan *post hoc* test disclosed that 11-hidroxi-erythravine, in the dose of 10 mg/kg and DZP increased expressively the time spent by the animals inside the illuminated compartment, when compared with the control group. The number of transitions between two compartments of the model has also been increased by the administration of 11-hidroxi-erythravine, with the dose of 3 mg/kg (P < 0.05).

**Table 8 - Effect (mean + EPM) of the acute treatment with 11-hidroxi-erythravine in house mouse submitted to the model of the clearly-dark transition (n = 8-10).**

| ***Treatments*** | ***Time spent in illuminated compartment*** | ***Number of transitions between two compartments*** |
|---|---|---|
| *SALINE* | *72.33±11.27* | *15.58±2.48* |
| *DZP (2 mg*/*kg)* | *130.00±16.76** | *93.92±1.34* |
| *11-OH (3 mg*/*kg)* | *98.67±13.35* | *22.67±2.66** |
| *11-OH (10 mg*/*kg)* | *927.22±21.83** | *94.00±1.93* |

| | | |
|---|---|---|
| * p < 0,05 versus Saline (Duncan *post hoc* Test) | | |

### 3) ERYTHRARTINE

The achieved effects on the transition model clear-dark with erythrartine could be seen by the data presented on Table 9.

The one route ANOVA showed a signified effect in the measure of time spent by the animals on the illuminated compartment (F(3,49) = 3.66; P < 0.01). This effect, according the Duncan test (P < 0.05), was observed only for the animals group treated with DZP (2 mg/kg). The one route ANOVA, however, did not show signified effect of the treatment in the measure of the transitions between the two compartments of the model (F(3,49) = 1.19; P < 0.31).

**Table 9 - Effect (media + EPM) of the acute treatment with erythrartine on mice submitted to the transition model clear-dark (n = 8-9).**

| ***Treatment*** | ***Spent time on the illuminated compartment*** | ***Transitions between the two compartments*** |
|---|---|---|
| *SALINE* | *70.59±6.48* | *16.65±1.67* |
| *DZP (2 mg*/*kg)* | *140.67±18.59** | *17.42±2.40* |
| *ERIT (3 mg*/*kg)* | *67.67±8.33* | *93.00±9.56* |
| *ERIT (10 mg*/*kg)* | *83.42±14.17* | *14.25±1.59* |

| | | |
|---|---|---|
| p < 0,05 versus Saline (Duncan *post hoc* Test) | | |

Three erythrinics alkaloids were isolated from the crude hydroalcoholic extract of *Erythrina mulungu,* the erhytrartine, the erhytravine and 11-OH-erhytravine. Previously, Sarragioto et al. (1981) had already reported the presence of erythratine, *N*-óxido-erhytrartine, erhysotrine and *N*-óxido-erysothrine on the methanol crude extract of *E*. *mulungu.* However, by this bidimensional test of gHMQC, the values attributed to the carbons C-1 and C-2 coupling constants have been amended by the present work.

The Erythravine was already isolated from the seeds extracts of *E*. *folkersii* (MILLINGTON *et al.,* 1973) and of *E*. *cochleata* (CHAWLA *et al.,* 1985). For the first time on the literature it is being reported the isolation of the 11-OH-erhytravine.

The results of the present invention show that the rich alkaloids purified fraction of *E. mulungu* it presents anxiolytic effect on the anxiety animal model of Labyrinth in high T. It were observed anxiolytic effect of the crude extract of *E. mulungu* as at the inhibitory avoidance as at the scape of the open arm of Labyrinth in high T. The doses of 100, 200 and 400 *mg*/*kg* acutely administrated damaged the acquisition of the inhibitory avoidance of the open arms. These results confirm the recents evidences which demonstrated the anxiolytic effects of the *E. mulungu* extract, also with the dose of 200 mg/kg, in the measure of avoidance in Labyrinth in high T for all mice (ONUSIC *et al.,* 2002). In this same work, the anxiolytic effect of the E. mulungu extract was also related with the clear-dark transition model (ONUSIC *et al.*, 2002). It was observed that the doses of 200 and 400 mg/kg, when acutely administrated in mice, increase the transitions between the two model compartments and the spent time by the animals at the illuminated compartment.

Considering that some compounds used on the pharmacotherapy for the treatment of the generalized anxiety, as the BDZ, diazepam, and the serotonergic receptors agonist of the type A, the buspirone (LADER, 1998; DAVIDSON JR., 2001; RICKELS *et al.,* 2001), it presents an anxiolytic effect in the measure of inhibitory avoidance of open arm of Labyrinth in high T. (GRAEFF *et al.,* 1993; VIANA *et al.,* 1994; GRAEFF *et al.,* 1998; CARVALHO NETTO e NUNES DE SOUZA, 2004) and on the clear-dark transition model (MERLO PICH e SAMANIN, 1989; CHAULOFF *et al.,* 1997; HASCÖSET e BOURIN, 1998), some authors suggests that these tests presents prevision validity for the generalized anxiety study (GRAEFF *et al.,* 1993; VIANA *et al.,* 1994; GRAEFF *et al.,* 1998; GRAEFF e ZANGROSSI, 2002; BOURIN e HASCÖSET, 2003).

The results obtained with the **F2,** a rich erythrinics alkaloids purified fraction as major substances, demonstrated anxiolytic effect effect in the measure of inhibitory avoidance of open arm. The acute administration of **F2,** at the doses of 3, 6, 10 e 17 mg/kg, damaged the acquisition of inhibitory avoidance, similarly to the used standard drug, the diazepan, indicating an anxiolytic effect. These results reinforce the conclusion that the observed anxiolytic effect with the crude extract is related to the presence of the erythrinics alkaloids in its composition. This can be confirmed by the realized tests with the isolated alkaloids on the realized experiments with the clear-dark transition model.

The clear-dark transition test, a classic anxiety animal model for the gabaergic and serotonergic drugs study was used for the valuation of the anxiolytic effect of the erythrartine, erythravine and 11-OH-erhytravine.

It was observed anxiolytic effect of the standardized crude extract (100 and 200 mg/kg) and of the alkaloids erythravine (3 and 10 mg/kg) and 11-hidroxi-erhytravine (10 mg/kg) on the measure of the spent time by the animals at the illuminated compartment. Positive results in this model with the *E*. *mulungu* extract had been reported recently (ONUSIC *et al.,* 2002). It was observed that the doses of 200 and 400 mg/kg when acutely administrated at mice increased the transitions between the two compartments of the model and the spent time by the animals at the illuminated compartment.

At the present invention it was also observed that the 11-hidroxi-erhytravine administrated at the lower dose (3 mg/kg) increased the transitions between the two compartments of the model. This increase of the transitions can be considered as an anxiolytic effect, a result of the behavior of lack of inhibition and not as a stimulant effect of the animal's activity, a time that did not have an increase of the response on the locomotion activity test at the arena. It can be concluded that:
- The present invention described new results obtained with the the Labyrinth in high T, which demonstrated the anxiolytic effect as the *Erythrine Mulungu* hydroalcoholic extract (EM) (100 - 400 mg/kg) as the isolated alkaloids from it, 11-OH-erhytravine, erythravine and erythrartine (3 and 10 mg/kg) when administrated by oral via;
- It was demonstrated with the described results - obtained by the arena test - that the EM and the tested substances does not modify the animal's locomotion activity;
- The results showed that a crude extract containing, at least, 0.1 % of total erythrinics alkaloids and a purified fraction (F2) containing, at least, 20% of total erythrinics alkaloids and 10% of the erythrinic alkaloid erythravine, beyond other alkaloids at minor concentrations and other polar and apolar substances also presented an anxiolytic effect at the LTE (3 - 30mg/kg; v.o), without change the house mouse locomotion activity;
- The obtained results with the clear-dark model transition, a classical instrument of *in vivo* pharmacological *screening* demonstrated that the 11-OH-erhytravine and the erythravine presented an anxiolytic effect when are administrated by oral via at the doses of 3 and 10 mg/kg;
- A pilot acute toxicity test (7 days, oral via) demonstrated that the crude extract did not cause the male or female house mouse death with the used doses(200 - 3600 mg/kg), revealing therefore of low toxicity.

Therefore, the data of the present document corroborate previous results indicating that the isolated alkaloids of *Erythrine mulungu,* i.e., 11-OH-erhytravine, erythravine and erythrartine are the main actives related to the anxiolytic effect of the hydroalcoholic extract and they keep this activity even when are administrated in crude extract or purified fraction (F2), which explain the wide popular utilization of this plant as a sedative, beyond to provide the preparations of pharmaceutical compositions standardized and with a suitable pharmacological action to the requirements of specifics therapies, without confront the common problems regarding the use of the plant at the *in natura* form. Beyond this, it can be said that the observed anxiolytic effect of the crude extract at previous studies is a result of the presence of the erythrinics alkaloids in its constitution that can be observed either by the tests made with isolated alkaloid or by the rich alkaloids purified fraction.

## Claims

1. A purified plant **fraction** rich in alkaloids for use as an anxiolytic agent, wherein said purified plant **fraction** contains at least 20% (w/w) of total erythrinic alkaloids and at least 10% (w/w) of erythravine and the plant is from the *Erythrine* genus.

2. The purified plant **fraction** according to claim 1 wherein the plant is *Erythrina Mulungu.*

3. The purified plant **fraction** according to any one of previous claims being orally administered.

## Patentansprüche

1. Gereinigte Pflanzenfraktion, die reich an Alkaloiden ist, für die Verwendung als Anxiolytikum, wobei die gereinigte Pflanzenfraktion insgesamt mindestens 20 % (Gew./Gew.) erythrinische Alkaloide und mindestens 10 % (Gew./Gew.) Erythravin enthält und die Pflanze aus der Gattung *Erythrina* stammt.

2. Gereinigte Pflanzenfraktion nach Anspruch 1, wobei es sich bei der Pflanze um *Erythrina Mulungu* handelt.

3. Gereinigte Pflanzenfraktion nach einem der vorhergehenden Ansprüche, die oral verabreicht wird.

## Revendications

1. Fraction végétale purifiée riche en alcaloïdes destinée à être utilisée en tant qu'agent anxiolytique, dans laquelle ladite fraction végétale purifiée contient au moins 20 % (p/p) d'alcaloïdes érythriniques totaux et au moins 10 % (p/p) d'érythravine et la plante appartient au genre *Erythrine.*

2. Fraction végétale purifiée selon la revendication 1, dans laquelle la plante est *Erythrina Mulungu.*

3. Fraction végétale purifiée selon l'une quelconque des revendications précédentes, ladite fraction végétale étant administrée par voie orale.
